(19) Europäisches Patentamt / European Patent Office / Office européen des brevets

(11) **EP 4 313 403 B1**

(12) **FASCICULE DE BREVET EUROPEEN**

(45) Date de publication et mention
de la délivrance du brevet:
**26.02.2025 Bulletin 2025/09**

(21) Numéro de dépôt: **22735497.4**

(22) Date de dépôt: **21.04.2022**

(51) Classification Internationale des Brevets (IPC):
**B01J 23/12** *(2006.01)*    **C10L 3/08** *(2006.01)*
**B01J 37/03** *(2006.01)*    **B01J 37/02** *(2006.01)*
**C12P 5/02** *(2006.01)*    **B01J 23/83** *(2006.01)*
**B01J 23/755** *(2006.01)*    **C07C 1/12** *(2006.01)*
**B01J 35/61** *(2024.01)*    **B01J 37/08** *(2006.01)*

(52) Classification Coopérative des Brevets (CPC):
(C-Sets disponibles)
**B01J 23/755; B01J 23/12; B01J 23/83;**
**B01J 35/612; B01J 37/0201; B01J 37/088;**
**C07C 1/12; C10L 3/08;** Y02E 60/36; Y02P 20/133
(Cont.)

(86) Numéro de dépôt international:
**PCT/FR2022/050745**

(87) Numéro de publication internationale:
**WO 2022/229541 (03.11.2022 Gazette 2022/44)**

(54) **PROCÉDÉ DE CONVERSION DU CO2 EN MÉTHANE**

VERFAHREN ZUR UMWANDLUNG VON CO2 IN METHAN

METHOD FOR CONVERTING CO2 INTO METHANE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **29.04.2021 FR 2104508**

(43) Date de publication de la demande:
**07.02.2024 Bulletin 2024/06**

(73) Titulaire: **Orano Chimie-Enrichissement
92320 Châtillon (FR)**

(72) Inventeurs:
• **TRUONG-PHUOC, Lai
67200 STRASBOURG (FR)**
• **NHUT, Jean-Mario
67115 PLOBSHEIM (FR)**
• **GIAMBASTIANI, Giuliano
50019 FLORENCE (IT)**
• **PHAM-HUU, Cuong
67100 STRASBOURG (FR)**

• **JOURDAN, Alex
84100 ORANGE (FR)**

(74) Mandataire: **Brevalex
Tour Trinity
1 B Place de la Défense
92400 Courbevoie (FR)**

(56) Documents cités:
**US-A- 4 032 556**

• **BERRY FRANK J. ET AL: "Nickel-uranium oxide
catalysts: characterisation and evaluation for
methanation", vol. 100, no. 1, 1 July 1993
(1993-07-01), AMSTERDAM, NL, pages 131 - 143,
XP055865499, ISSN: 0926-860X, Retrieved from
the Internet <URL:https://www.sciencedirect.
com/science/article/pii/0926860X93801216/pdf?
md5=c41bdac9f1b3c6d941599d6b81be019d&
pid=1-s2.0-0926860X93801216-main.pdf>
[retrieved on 20211125], DOI: 10.1016/
0926-860X(93)80121-6**

EP 4 313 403 B1

(52) Classification Coopérative des Brevets (CPC):
(Cont.)

C-Sets
**C07C 1/12, C07C 9/04**

## Description

La présente invention se rapporte à un procédé de conversion catalytique du dioxyde de carbone en présence d'hydrogène en méthane (procédé de méthanation) mettant en oeuvre un catalyseur à base de nickel dispersé sur un support à base d'oxyde d'uranium.

## ÉTAT DE LA TECHNIQUE

La production d'énergie durable, combinée à des pratiques de consommation modérées, représentent un défi pour notre civilisation. Au cours de cette dernière décennie, l'augmentation continue de la demande énergétique mondiale et la prise de conscience collective de la problématique du réchauffement climatique ont conduit au développement de moyens de production d'énergie électrique à partir de sources renouvelables.

Cette intégration massive de sources d'énergie renouvelables dans le paysage énergétique se heurte toutefois à la problématique de la gestion des réseaux électriques. La nature intermittente et localisée de la plupart de celles-ci, notamment de l'éolien et du solaire, compliquent à tout instant l'équilibrage de ces réseaux, entre la production et la demande en énergie électrique. À cela s'ajoute la nécessité de pouvoir gérer le stockage du surplus d'électricité de manière à pouvoir en bénéficier aisément en cas de chute en rendement des moyens de production d'énergie renouvelable.

Les moyens de stockage actuels ne permettent de stocker que des quantités limitées d'énergie et ne peuvent donc répondent qu'à des besoins qui s'étendent sur de courtes durées, quelques jours tout au plus.

Depuis quelques années, l'idée d'un moyen de stockage alternatif aux technologies traditionnelles a émergé et propose d'utiliser les énergies sous forme chimique. Ce moyen de stockage, appelé *Power-to-Gas*, propose ainsi de convertir l'énergie électrique en gaz, par exemple de l'hydrogène ou du méthane, qui est utilisé comme vecteur de stockage. Ce stockage chimique a l'avantage qu'il peut être maintenu sur des périodes longues comparées à celles des systèmes mis en oeuvre à l'heure actuelle.

La conversion de l'énergie électrique en gaz offre par ailleurs de nombreuses possibilités d'utilisation finale de cette énergie, comme le chauffage domestique, l'usage industriel ou encore la mobilité des personnes.

Le procédé *Power-to-Gas* consiste notamment à mettre en oeuvre une ou deux étapes de conversion selon le gaz de stockage choisi. Ce vecteur peut être, soit l'hydrogène, produit par l'électrolyse de l'eau alimentée par de l'énergie solaire ou éolienne, soit le méthane, produit dans une seconde étape dite de méthanation qui réalise la conversion d'hydrogène et du dioxyde de carbone en méthane. En plus de permettre la valorisation du $CO_2$ considéré comme un sous-produit de l'industrie, la réalisation de cette étape supplémentaire permet de limiter les contraintes et le coût de l'adaptation des stockages, de la distribution et de l'utilisation finale du vecteur énergétique.

La réaction de méthanation permet de convertir le $CO_2$ en présence d'hydrogène en méthane et de l'eau (Equation 1).

$$CO_2 + 4H_2 \rightarrow CH_4 + 2H_2O \qquad \text{Équation 1.}$$

Cette réaction est fortement exothermique et dégage une chaleur importante ($\Delta H^\circ_{298K} = -165,0$ kJ/mol). D'après le principe de Le Chatelier, la formation de méthane est favorisée à une faible température et sous une grande pression. Cependant, la molécule de $CO_2$ est une molécule linéaire stable, composée de deux liaisons doubles O=C, d'où la nécessité d'apporter un excès d'énergie pour activer cette molécule et d'employer un catalyseur pour s'affranchir des limitations cinétiques importantes de cette réaction.

De nombreuses recherches menées pour développer des catalyseurs permettant de diminuer la barrière d'énergie d'activation de cette réaction ont montré que des catalyseurs à base de métaux nobles sont particulièrement actifs mais leurs prix élevés constituent une limite à leurs utilisations. Il ressort que les catalyseurs à base de Ni dispersé sur un support sont les plus prometteurs pour la méthanisation du $CO_x$ (x = 1, 2) en raison de leurs bonnes performances catalytiques et de leur prix relativement bas. Les supports des catalyseurs à base de nickel couramment utilisés sont des oxydes tels que $Al_2O_3$, $SiO_2$, $TiO_2$, $ZrO_2$ et $CeO_2$. Parmi eux, $Ni/SiO_2$ et $Ni/Al_2O_3$ ont été largement étudiés en raison de leurs bonnes activités initiales mais souffrent, lorsqu'ils sont mis en oeuvre à haute température, de phénomènes de désactivation dûs au frittage des particules de la phase active (réduisant le nombre de sites actifs) et à des dépôts importants de carbone (coke) bloquant ainsi l'accès des réactifs aux sites actifs. Actuellement des catalyseurs à base de Ni supporté sur un support $Al_2O_3$ sont disponibles auprès de fabricants tels que Johnson Matthey, Haldor-Topsøe ou Clariant-Süd Chemie.

On connaît également dans l'état de la technique la publication de Berry et al. (Applied Catalysis A: General 100 (1993) 131-143) qui s'intéresse à des catalyseurs à base de Ni et d'oxyde d'uranium utiles pour la réaction de méthanation du $CO_2$. Les catalyseurs sont préparés par évaporation d'une solution aqueuse contenant du nitrate de nickel et du nitrate d'uranyle jusqu'à la formation d'un résidu visqueux qui se solidifie à température ambiante. Le solide ainsi obtenu est ensuite calciné sous air à une température de 1000 °C.

Un but de la présente invention est de proposer un procédé de méthanation du $CO_2$ qui réponde à plusieurs critères, notamment en termes de taux de conversion du $CO_2$, de sélectivité en $CH_4$, de productivité et qui peut être opéré notamment à des températures inférieures à 350 °C et, de préférence, inférieures à 300 °C, voire inférieures à 260 °C.

## RÉSUMÉ DE L'INVENTION

L'invention se rapporte donc à un procédé de conversion du $CO_2$ en méthane dans lequel :

- on met en contact de l'hydrogène et une charge gazeuse comprenant du $CO_2$ dans au moins un réacteur de méthanation comprenant un lit de catalyseur à une température dans le lit catalytique comprise entre 160 °C et 550 °C, à une pression comprise entre 0,1 MPa et 1 MPa, avec une vitesse spatiale horaire de gaz comprise entre 10 $m^3$/kg/h et 50 $m^3$/kg/h et avec un rapport molaire $H_2/CO_2$ compris entre 1 et 8 ;
- le catalyseur contient du Ni métal déposé sur un support en oxyde d'uranium de formule $UO_{2+x}$, avec x étant compris entre 0,01 et 0,6, et dont la teneur massique en nickel est comprise entre 5 % et 40 % de nickel métal par rapport à la masse totale du catalyseur ; et dans lequel :

  - le catalyseur est préparé par un procédé comprenant les étapes a) à c) suivantes :

    a) on imprègne un précurseur de support constitué essentiellement d'un oxyde d'uranium (IV) et/ou d'uranium (VI) avec une solution contenant un précurseur de nickel et un solvant polaire ;
    b) on calcine sous air et à une température d'au moins 250 °C le précurseur de support imprégné ; et
    c) on réduit le précurseur de support imprégné et calciné sous hydrogène à une température d'au moins 300 °C.

De manière surprenante, la demanderesse a constaté que la mise en oeuvre d'un catalyseur comprenant du nickel métal dispersé sur un support à base d'oxyde d'uranium $UO_{2+x}$ avec x étant compris entre 0,01 et 0,6 présente une activité exacerbée pour la conversion du $CO_2$ en méthane, de sorte que le procédé peut être conduit à des températures moins élevées que celle de l'art antérieur, tout en maintenant un rendement et une sélectivité en $CH_4$ élevés, c'est-à-dire respectivement supérieurs à 60 % et proches de 100 %.

On entend par le vocable « constitué essentiellement d'un oxyde d'uranium (IV) et/ou d'uranium (VI) » désigner un support dont la teneur oxyde d'uranium (IV) et/ou d'uranium (VI) est d'au moins 90 % en masse.

La mesure de la température du lit catalytique peut être réalisée par toute méthode connue de l'homme du métier comme par exemple au moyen d'un ou plusieurs thermocouples disposés dans ledit lit ou par pyrométrie laser.

Selon des modes de réalisation particuliers, le procédé comprend l'une ou plusieurs des caractéristiques suivantes, prise(s) isolément ou selon toutes les combinaisons techniquement possibles.

Le procédé de préparation du catalyseur peut comprendre, avant l'étape b) de calcination, une étape dans laquelle on sèche le précurseur de support imprégné à une température inférieure à 200 °C afin d'éliminer notamment le solvant de la solution d'imprégnation. De préférence, la solution contient un solvant polaire dans lequel le précurseur de nickel est solubilisé. De manière préférée, il s'agit d'une solution aqueuse contenant le précurseur de nickel.

De préférence, l'étape b) de calcination est réalisée à une température d'au moins 300 °C pendant au moins 1 heure, de préférence pendant au moins 2 heures.

De préférence, l'étape c) de réduction est conduite à une température d'au moins 350 °C pendant au moins 1 heure, de préférence pendant au moins 2 heures. L'hydrogène nécessaire à la réduction est apporté sous forme d'hydrogène pur. Cette étape de réduction permet non seulement de convertir l'oxyde d'uranium (VI) ou l'oxyde d'uranium (IV) et (VI) (e.g. $U_3O_8$) en $UO_{2+x}$ et de former du nickel métal.

Le précurseur de support à base d'oxyde d'uranium (IV) et/ou d'uranium (VI) peut être choisi parmi l'$UO_2$, l'$UO_3$, l'$UO_4$ et l'$U_3O_8$. De préférence, le précurseur de support est de l'$U_3O_8$.

Selon l'invention, le précurseur de support peut prendre toute forme. Par exemple, le précurseur de support présente une morphologie sous forme d'extrudés cylindriques ou multilobés, de sphères ou d'une poudre de granulométrie variable. Selon un mode de réalisation, lorsque le précurseur du support est une poudre, il peut avantageusement être mis en forme (e.g. billes ou extrudés) après l'étape d'imprégnation du précurseur de nickel.

Pour l'étape d'imprégnation, le précurseur de nickel peut être choisi parmi l'hydroxyde, l'hydroxycarbonate, le carbonate et le nitrate de nickel, avec une préférence pour le nitrate de nickel. L'étape a) d'imprégnation du précurseur de nickel peut être réalisée selon les méthodes d'imprégnation à sec ou en excès.

L'étape c) peut être réalisée de manière *ex situ* ou *in situ*, c'est-à-dire directement dans le réacteur de méthanation.

Pour la mise en oeuvre du procédé de conversion, l'hydrogène et le $CO_2$ sont envoyés séparément dans le réacteur de méthanation, par exemple selon une direction descendante. Alternativement, les deux réactifs gazeux sont préalablement mélangés avant d'être envoyés dans le réacteur de méthanation.

Le réacteur de méthanation est un réacteur de type adiabatique, isotherme ou hybride. De préférence, le réacteur de méthanation est un réacteur adiabatique à lit fixe ou fluidisé de catalyseur. Le chauffage du réacteur peut être opéré par toute méthode connue de l'homme du métier par exemple au moyen d'une résistance disposée dans le lit catalytique, d'un système d'échangeur de chaleur interne (type serpentin) ou externe.

Selon un mode de réalisation, lorsque le lit de catalyseur est un lit fixe, on soumet le lit catalytique à un champ électromagnétique alternatif de manière à chauffer le lit catalytique par induction.

Afin de favoriser le chauffage par induction, le lit de catalyseur comprend avantageusement un suscepteur, c'est-à-dire un élément qui, lorsqu'il est soumis à un champ électromagnétique alternatif, est capable de convertir l'énergie électromagnétique en chaleur et de la communiquer au catalyseur. Ceci peut être le résultat de pertes par hystérésis et/ou de courants de Foucault induits dans le suscepteur qui dépendent notamment des propriétés électriques et magnétiques du matériau du suscepteur. Les pertes par hystérésis se produisent dans les suscepteurs ferromagnétiques ou ferrimagnétiques et résultent de la commutation des domaines magnétiques à l'intérieur du matériau lorsque ce dernier est soumis à l'influence d'un champ électromagnétique alternatif. Des courants de Foucault peuvent être induits si le suscepteur est électriquement conducteur. Dans le cas d'un suscepteur ferromagnétique ou ferrimagnétique électriquement conducteur, de la chaleur peut être générée à la fois par des courants de Foucault et par des pertes par hystérésis. Dans ce cas, le chauffage est réalisé essentiellement en surface du suscepteur qui peut transmettre la chaleur à la surface du catalyseur avec lequel il est en contact. Par exemple, le suscepteur peut être choisi parmi des matériaux carbonés/-graphitiques, des métaux ou alliages de métaux qui ne sont pas réactifs pour la réaction visée tels que, par exemple, l'aluminium, le fer, le cuivre, le bronze, l'acier inoxydable, l'acier inoxydable ferritique, l'acier inoxydable martensitique et l'acier inoxydable austénitique. Le suscepteur peut être soit en contact direct avec le catalyseur soit séparé du catalyseur par une paroi non isolante thermiquement de manière à permettre un transfert rapide et homogène de la chaleur au catalyseur.

De façon avantageuse, afin de répondre aux enjeux de la transition énergétique, on opère le procédé selon l'invention avec une source d'énergie renouvelable afin de stocker cette dernière sous forme chimique.

Selon un mode de réalisation, le procédé de conversion met en oeuvre un réacteur à lit fixe de catalyseur et dans lequel on fixe la vitesse spatiale horaire gazeuse à une valeur d'au moins 15 $m^3$/kg/h de manière à maintenir une température dans le lit catalytique à au moins 200 °C, moyennant quoi on opère la réaction de conversion sans apport de chaleur externe.

L'invention concerne également un catalyseur de méthanation du $CO_2$ comprenant du nickel métal déposé sur un support en oxyde d'uranium de formule $UO_{2+x}$ avec x étant compris entre 0,01 et 0,6 et dans lequel la teneur massique en nickel métal est comprise entre 5 % et 40 % de Ni par rapport à la masse totale du catalyseur. De préférence, le catalyseur selon l'invention est constitué de nickel métal déposé sur un support en oxyde d'uranium de formule $UO_{2+x}$ avec x étant compris entre 0,01 et 0,6.

La teneur en nickel métal est de préférence comprise entre 10 % et 20 % en masse de nickel par rapport à la masse totale de catalyseur.

Enfin, l'invention se rapporte à un procédé de préparation d'un catalyseur de méthanation du $CO_2$ comprenant du nickel métal déposé sur un support en oxyde d'uranium de formule $UO_{2+x}$ avec x étant compris entre 0,01 et 0,6 et dans lequel la teneur massique en nickel métal est comprise entre 5 % et 40 % de Ni par rapport à la masse totale du catalyseur, lequel procédé comprend les étapes a) à c) suivantes :

a) on imprègne un précurseur de support constitué essentiellement d'un oxyde d'uranium (IV) et/ou d'uranium (VI) avec une solution contenant un précurseur de nickel et un solvant polaire ;
b) on calcine sous air et à une température d'au moins 250 °C le précurseur de support imprégné ;
c) on réduit sous hydrogène à une température d'au moins 300 °C, le précurseur de support imprégné et calciné.

## DESCRIPTION DETAILLÉE DE L'INVENTION

### Description des charges gazeuses

Le procédé selon l'invention permet de traiter une charge gazeuse présentant une teneur volumique en $CO_2$ supérieure à 30 %, de préférence supérieure à 50 % et encore mieux supérieure à 90 %. Alternativement, la charge gazeuse contenant du $CO_2$ peut être en mélange avec du méthane qui peut être à une teneur en volume d'au plus 50 %.

Cette charge gazeuse de $CO_2$ est, par exemple, un effluent gazeux issu d'une unité de méthanisation de la biomasse, d'une unité de gazéification, d'une unité de raffinage du pétrole ou d'une cimenterie. Le $CO_2$ peut être également provenir des unités de capture de $CO_2$.

Quant à la charge gazeuse d'hydrogène ($H_2$), elle peut être obtenue à partir de l'électrolyse de l'eau ou provenir d'une unité de reformage catalytique de coupes pétrolières lourdes.

Selon un mode de réalisation préféré qui s'inscrit dans le concept des procédés *Power-to-Gas* permettant de stocker des énergies renouvelables sous forme chimique, l'hydrogène est produit dans des unités électrolyse de l'eau alimentées par des centrales solaires ou éoliennes.

De préférence, la teneur volumique en $H_2$ de cette charge est supérieure à 90 % et, de préférence, supérieure à 95 %.

Selon l'invention, les charges de $CO_2$ et de dihydrogène peuvent être mélangées avant d'être envoyées dans le réacteur de méthanation (mode d'opération préférentiel) ou bien être distribuées séparément dans le réacteur de méthanation. On opère la réaction de conversion en présence d'un mélange gazeux dont le rapport molaire $H_2/CO_2$ est compris entre 1 et 8, de préférence compris entre 1 et 4, et plus préférentiellement égal à 4.

**Description du catalyseur de méthanation selon l'invention**

Le procédé de méthanation selon l'invention met en oeuvre un catalyseur hétérogène comprenant du nickel métallique supporté sur un oxyde d'uranium répondant à la formule $UO_{2+x}$ avec x étant compris entre 0,01 et 0,6.

Le nickel est présent dans le catalyseur à une teneur massique comprise entre 5 % et 40 % en Ni métal par rapport à la masse totale du catalyseur. De préférence, la teneur massique en Ni métal est comprise entre 10 % et 20 % par rapport à la masse totale du catalyseur.

Le catalyseur selon l'invention se distingue de celui de l'art antérieur décrit dans Applied Catalysis A: General 100 (1993) 131-143, en ce qu'il est notamment obtenu par imprégnation d'un précurseur du support avec une solution contenant un sel de nickel, puis calcination du support imprégné et enfin réduction sous hydrogène du produit de la calcination. De façon avantageuse, notamment afin de réduire le temps de traitement par calcination, le support imprégné est soumis à une étape de séchage avant l'étape de calcination.

Dans le cadre de l'invention, le précurseur de support à base l'oxyde d'uranium peut provenir de la filière nucléaire d'enrichissement de l'uranium naturel en uranium 235 ($U^{235}$), laquelle fournit de l'uranium dit « appauvri », c'est-à-dire dont la teneur massique en $U^{235}$ est inférieure à 0,7 %, généralement comprise entre 0,2 % et 0,4 %.

Dans une première étape du procédé de synthèse du catalyseur, on imprègne le précurseur de support à base d'oxyde d'uranium avec une solution contenant un sel soluble de nickel. La solution contient un solvant polaire, qui est de préférence de l'eau, dans lequel est dissous un sel de nickel qui peut être choisi parmi l'hydroxyde, l'hydroxycarbonate, le carbonate et le nitrate de nickel. La teneur en nickel de la solution d'imprégnation peut prendre n'importe quelle valeur mais, de préférence, cette valeur est inférieure à la saturation du sel dans le solvant utilisé. De préférence, on utilise une solution la plus concentrée en sel soluble de nickel tout en évitant la saturation de la solution d'imprégnation. Dans le cas où le catalyseur selon l'invention contient de fortes teneurs en Ni (e.g. supérieures à 20 %), on peut procéder par imprégnations successives du support avec optionnellement des étapes de séchage et de calcination intermédiaires.

Le précurseur du support peut se trouver sous forme d'extrudés de petit diamètre, cylindriques ou multilobés (trilobes, quadrilobes, etc.), de sphères, d'anneaux, de monolithes en structure de nid d'abeille ou sous forme d'une poudre.

La surface spécifique BET du précurseur est généralement comprise entre 1 $m^2/g$ et 10 $m^2/g$, de préférence entre 1 $m^2/g$ et 5 $m^2/g$. La surface spécifique est déterminée par porosimétrie à l'azote.

L'étape de mise en contact dudit précurseur de support avec une solution d'imprégnation contenant du nickel peut être réalisée soit par imprégnation en slurry, soit par imprégnation en excès, soit par imprégnation à sec, soit par tout autre moyen connu de l'Homme du métier. L'imprégnation à l'équilibre (ou en excès), consiste à immerger le support dans un volume de solution (souvent largement) supérieur au volume poreux du support en maintenant le système sous agitation pour améliorer les échanges entre la solution et le support ou catalyseur. Un équilibre est finalement atteint après diffusion des différentes espèces dans les pores du support. La maîtrise de la quantité d'éléments déposés est assurée, par exemple, par la mesure préalable d'une isotherme d'adsorption qui permet de relier la concentration des éléments à déposer contenus dans la solution à la quantité des éléments déposés sur le solide en équilibre avec cette solution.

L'imprégnation à sec consiste, quant à elle, à introduire un volume de solution d'imprégnation égal au volume poreux du support. L'imprégnation à sec permet de déposer sur un support ou catalyseur donné l'intégralité des additifs contenus dans la solution d'imprégnation.

L'étape d'imprégnation de la solution de nickel peut être avantageusement effectuée par une ou plusieurs imprégnations en excès de solution ou, de préférence, par une ou plusieurs imprégnations à sec.

Cette étape d'imprégnation peut être menée à une température comprise entre 18 °C et 50 °C, de préférence comprise entre 20 °C et 30 °C.

À l'issue de l'étape a), on peut avantageusement laisser maturer le support imprégné de manière à permettre une dispersion homogène de la solution d'imprégnation au sein du support. Toute étape de maturation est avantageusement réalisée à pression atmosphérique, à une température comprise entre 18 °C et 50 °C et, de préférence, à température ambiante. Généralement, une durée de maturation comprise entre 10 minutes et 48 heures et, de préférence, comprise entre 30 minutes et 6 heures, est suffisante.

Lorsque le précurseur de support est une poudre, après l'étape d'imprégnation du précurseur de nickel, celui-ci est avantageusement mis en forme, par exemple, par sphéronisation ou par extrusion.

Le précurseur de support après imprégnation est optionnellement soumis à une étape de séchage à une température inférieure à 200 °C, avantageusement comprise entre 50 °C et 150 °C, de préférence entre 70 °C et 150 °C, de manière très préférée entre 75 °C et 130 °C. L'étape de séchage est préférentiellement réalisée sous une atmosphère contenant de l'oxygène, de préférence sous air. L'étape de séchage peut être effectuée par toute technique connue de l'Homme du métier. Elle est avantageusement effectuée à pression atmosphérique ou à pression réduite. De manière préférée, cette étape est réalisée à pression atmosphérique. Elle est avantageusement effectuée en lit traversé en utilisant de l'air ou tout autre gaz chaud. De manière préférée, lorsque le séchage est effectué en lit fixe, le gaz utilisé est soit l'air, soit un gaz inerte comme l'argon ou l'azote. De manière très préférée, le séchage est réalisé en lit traversé sous air. De préférence, l'étape de séchage a une durée comprise entre 5 minutes et 15 heures, de préférence comprise entre 2 heures et 12 heures.

Il est à noter que les étapes d'imprégnation, de maturation et/ou séchage peuvent être répétées plusieurs fois de suite jusqu'à parvenir à la quantité souhaitée de nickel déposée sur le précurseur de support.

Le précurseur de support imprégné, éventuellement maturé et séché, est soumis ensuite à une étape de calcination sous atmosphère oxydante, de préférence sous air ou sous oxygène dilué et à une température d'au moins 250 °C et, de préférence, d'au moins 300 °C. Typiquement, on calcine le précurseur imprégné à une température comprise entre 300 °C et 500 °C sous air et pendant 1 heure à 5 heures.

La mise en oeuvre du catalyseur de méthanisation selon l'invention requiert qu'il soit ensuite activé dans une étape de réduction afin de convertir au moins une partie de formes oxydées du nickel générées lors de l'étape de calcination en nickel métal et de réduire l'oxyde d'uranium (VI) ou l'oxyde d'uranium (IV) et (VI) en $UO_{2+x}$. À cette fin, le catalyseur est mis en contact avec de l'hydrogène pur ou dilué à une température au moins égale à 300 °C, de préférence comprise entre 350 °C et 500 °C, pendant une durée d'au moins une heure et de préférence entre 2 heures et 5 heures. Cette activation par réduction peut être conduite de manière *ex situ* dans un réacteur de réduction dédié ou bien *in situ*, c'est-à-dire directement dans le réacteur de méthanation après son chargement dans ledit réacteur.

Le catalyseur activé présente généralement une surface spécifique BET, mesurée par une isotherme d'adsorption à l'azote, comprise entre 1 $m^2/g$ et 10 $m^2/g$ et, de préférence, comprise entre 2 $m^2/g$ et 6 $m^2/g$.

Le catalyseur selon l'invention a une excellente activité catalytique pour la méthanation du $CO_2$ qui se traduit par un rendement en $CH_4$ d'au moins 60 %, de préférence d'au moins 80 % et avec une sélectivité en $CH_4$ proche de 100 %.

**Mise en œuvre du procédé de méthanation selon l'invention**

Le procédé de méthanation du $CO_2$ selon l'invention consiste à mettre en contact, dans un réacteur, la charge gazeuse contenant majoritairement du $CO_2$, de l'hydrogène et le catalyseur décrit ci-avant. Dans le cadre de l'invention, la charge gazeuse contenant du $CO_2$ peut être mélangée avec un flux d'hydrogène et le mélange est ensuite envoyé dans le réacteur contenant le catalyseur.

La mise en contact avec le catalyseur peut être réalisée dans un réacteur adiabatique, isotherme ou hybride. Par « réacteur adiabatique », on désigne un réacteur qui n'échange pas de chaleur avec le milieu extérieur ; la chaleur dégagée par la réaction exothermique de méthanation sert alors à alimenter la réaction et la partie excédentaire de chaleur est évacuée par les effluents soutirés du réacteur.

Par « réacteur isotherme », on entend un réacteur qui est refroidi grâce à la circulation d'un fluide permettant de contrebalancer la libération locale de chaleur de réaction.

Enfin, on entend par « réacteur hybride » désigner un réacteur combinant les deux caractéristiques précitées dans lequel un flux de refroidissement est appliqué pour contrebalancer en partie le dégagement local de chaleur de la réaction mais dans lequel il existe encore un gradient de température significatif au sein du réacteur.

La mise en contact des réactifs gazeux avec le catalyseur selon l'invention est réalisée à une température dans le lit catalytique comprise entre 160 °C et 550 °C, de préférence comprise entre 180 °C et 350 °C.

Le procédé selon l'invention est opéré avec une vitesse spatiale horaire de gaz, rapport entre le débit de gaz en $m^3/h$ et la masse de catalyseur en kg, comprise entre 10 $m^3/kg/h$ et 50 $m^3/kg/h$, de préférence comprise entre 15 $m^3/kg/h$ et 30 $m^3/kg/h$ et avec un rapport molaire $H_2/CO_2$ compris entre 1 et 8 (mol/mol), de préférence compris entre 1 et 4 (mol/mol), voire compris entre 3 et 4 et encore mieux égal à 4.

La charge gazeuse peut être envoyée dans le réacteur selon une direction descendante ou ascendante, préférentiellement selon une direction descendante avec un soutirage des produits de réaction en fond du réacteur.

Le catalyseur selon l'invention peut être mise en oeuvre dans un réacteur en lit fixe ou en lit fluidisé.

Lorsque le réacteur est de type lit fixe, il peut comprendre une pluralité de tubes perforés dans lesquels est disposé le catalyseur avec éventuellement des éléments de garnissage. Alternativement, le lit catalytique fixe peut être délimité par des plateaux inférieur et supérieur perforés dont les diamètres correspondent au diamètre intérieur du réacteur et entre lesquels est disposé le catalyseur avec éventuellement des éléments de garnissage.

L'énergie thermique nécessaire à apporter dans le réacteur peut être fournie par toute méthode connue de l'homme du métier, notamment avec un système d'échangeur de chaleur interne (type serpentin) ou externe, par effet Joule, par micro-ondes et par chauffage inductif.

De manière inattendue, il a été observé que les catalyseurs selon l'invention sont susceptibles d'être chauffés sous l'action d'un champ électromagnétique alternatif.

Selon un mode de réalisation préféré, le réacteur de méthanation met en oeuvre un lit fixe de catalyseur qui est soumis à un champ électromagnétique alternatif qui provoque son échauffement sans contact avec la source d'énergie. Ce mode de chauffage permet de fournir l'énergie nécessaire à la réaction seulement au catalyseur tandis que les réactifs gazeux entrant et sortant du lit catalytique ne sont ni chauffés ni refroidis.

Selon l'invention, afin d'améliorer l'efficacité du chauffage par induction, le lit catalytique peut comprendre un mélange de catalyseur selon l'invention avec un matériau conducteur électrique (suscepteur) dont le rôle est de communiquer de la chaleur supplémentaire au catalyseur. L'emploi d'un suscepteur est préconisé lorsque le catalyseur présente une teneur massique en nickel inférieure à 15 % par rapport à la masse totale de catalyseur. À titre d'exemple, le matériau suscepteur peut être choisi parmi des matériaux carbonés/graphitiques, des métaux ou alliage de métaux qui ne sont pas réactifs pour la réaction visée tels que, par exemple, l'aluminium, le fer, le cuivre, le bronze, l'acier inoxydable, l'acier inoxydable ferritique, l'acier inoxydable martensitique et l'acier inoxydable austénitique. Selon un mode de réalisation alternatif, le suscepteur est séparé du lit catalytique au travers d'une paroi non isolante thermiquement autorisant le transfert de la chaleur au catalyseur.

Ce mode de mise en oeuvre par chauffage par induction présente plusieurs avantages :

- un réglage précis de la température au sein du lit catalytique ;
- une régulation extrêmement rapide, tant en montée qu'en descente, de la température dans le lit catalytique ;
- le réactif entrant n'étant pas chauffé, il contribue ainsi à extraire de manière efficace l'énergie thermique dégagée par la réaction ;
- dans la mesure où seul le catalyseur est chauffé et non son environnement immédiat, l'évacuation et le maintien de la température dans le lit catalytique sont favorisés, ce qui permet de minimiser les risques d'emballement thermique ;
- un rendement énergétique amélioré ;
- la vapeur d'eau générée par la réaction peut être en partie condensée dans l'espace vide se trouvant entre les grains de catalyseur, car seul le solide est chauffé, et favoriser ainsi la conversion des réactifs en réduisant les problèmes d'adsorption compétitifs à la surface du catalyseur.

Pour opérer le chauffage par induction, le réacteur comprend un dispositif inducteur capable de générer un champ électromagnétique. Le dispositif inducteur peut être disposé à l'intérieur du réacteur de manière à encercler le lit catalytique de sorte que le champ magnétique qu'il génère soit essentiellement perpendiculaire à l'épaisseur du lit catalytique. Selon un second mode de réalisation, le dispositif inducteur est disposé au niveau du lit catalytique mais à l'extérieur ou dans la paroi du réacteur. Ce second mode de réalisation présente l'avantage que l'inducteur est découplé de l'environnement chimique et permet ainsi un pilotage plus aisé de l'inducteur. Toutefois, dans ce second mode de réalisation, on privilégiera l'utilisation d'un réacteur fabriqué en un matériau non conducteur électrique tel que, par exemple, en verre ou en céramique. L'ensemble peut être protégé par une autre enceinte externe.

Le dispositif d'inducteur est, par exemple et de manière non limitative, une bobine d'induction hélicoïdale s'étendant sur l'épaisseur du lit catalytique fixe ou bien une pièce formant un anneau dont la hauteur correspondant sensiblement à l'épaisseur du lit catalytique.

Il est à noter que le catalyseur selon l'invention est particulière adapté à ce type de chauffage par induction car il possède une densité élevée, de l'ordre de 6 g/cm$^3$ à 12 g/cm$^3$ et une surface spécifique relativement faible, de l'ordre de 2 m$^2$/g à 6 m$^2$/g.

Un autre avantage apporté par un chauffage par induction est de permettre une séparation aisée de l'eau formée au cours de la conversion du $CO_2$ et cela sans avoir recours à une unité de condensation dédiée utilisant un système d'échangeur de chaleur. En effet, dans la mesure où seul le lit catalytique est soumis à un chauffage, l'effluent gazeux contenant un mélange de méthane et d'eau subit un refroidissement brusque en sortie du réacteur (phénomène de « quench » selon la terminologie anglo-saxonne) provoquant alors une condensation au moins partielle de l'eau à l'état liquide. L'effluent qui est soutiré du réacteur peut être envoyé dans un ballon de séparation afin de séparer une phase gazeuse contenant le méthane en mélange avec éventuellement du $CO_2$ et de l'hydrogène n'ayant pas réagi et une phase liquide constituée d'eau.

En raison de l'activité catalytique exacerbée du catalyseur selon l'invention, les inventeurs ont constaté que, de manière surprenante, le procédé de conversion du $CO_2$ peut être conduit dans des conditions opératoires spécifiques selon un mode dit en « auto-méthanation », dans lequel la chaleur dégagée au sein du lit catalytique est suffisante pour entretenir la réaction sans besoin d'un apport extérieur en énergie.

Ce mode de fonctionnement en « auto-méthanation » est possible lorsque on met en oeuvre un réacteur avec un lit fixe de catalyseur, avec une vitesse spatiale horaire de gaz d'au moins 15 m$^3$/kg/h en présence d'un mélange gazeux ayant un ratio molaire $H_2/CO_2$ compris entre 3 et 4, de préférence égal à 4, et avec une température au sein du lit catalytique comprise entre 200 °C et 450 °C, de préférence comprise entre 220 °C et 300 °C, après stabilisation des flux thermiques dans le lit catalytique.

Afin de limiter les risques d'emballement thermique dans le réacteur, plusieurs mesures peuvent être prises seules ou en combinaison. Ainsi on peut choisir de :

i) travailler sur la dilution du flux réactionnel : lors du démarrage de la réaction, un flux plus dilué (par ajout d'un gaz inerte) pourrait être utilisé afin de réduire la chaleur dégagée par la réaction, puis, lorsque la température optimale de la réaction est atteinte, on augmente la concentration des réactifs étape par étape afin favoriser la conversion des réactifs tout en maintenant un équilibre entre chaleur dégagée par la réaction et la chaleur évacuée pour ne pas provoquer un emballement thermique brusque difficile à contrôler ;
ii) remplacer le chauffage par effet Joule avec des fortes inerties thermiques en termes de régulation par un chauffage inductif comme décrit plus haut ;
iii) appliquer un refroidissement forcé du réacteur au moyen d'une circulation d'un fluide de refroidissement pour évacuer la chaleur et maintenir ainsi la température de réaction.

## BRÈVE DESCRIPTION DES FIGURES

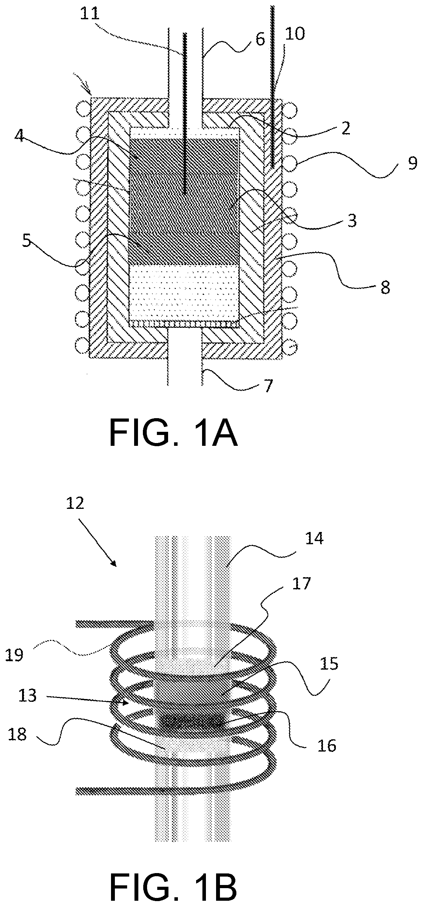

La figure 1A illustre schématiquement le montage ayant été utilisé par les Inventeurs pour réaliser les tests catalytiques sous chauffage indirect par effet Joule.
La figure 1B illustre schématiquement le montage permettant un chauffage par induction du lit catalytique.

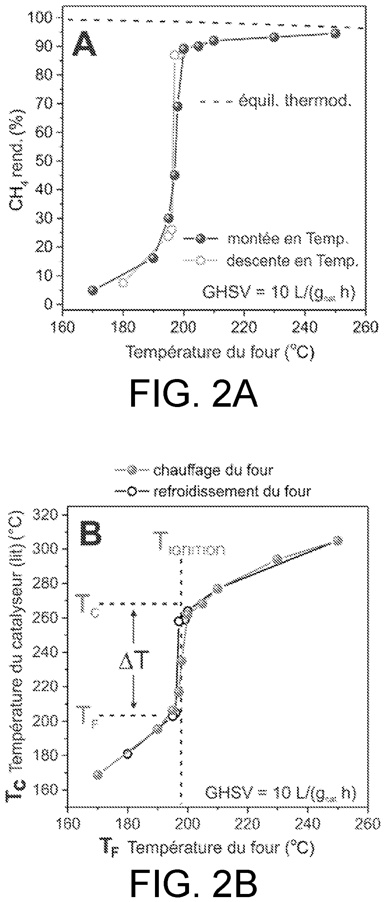

Les figures 2A à 2C représentent respectivement le rendement en méthane en fonction de la température du four, notée $T_F$, la variation de la température du lit catalytique en fonction de la température du four et le rendement en méthane en fonction de la température du lit catalytique obtenus lors de tests avec un catalyseur Ni10/UO$_{2+x}$ en présence d'un mélange gazeux de rapport molaire $H_2/CO_2$ de 4, avec une vitesse spatiale horaire de gaz de 10 L/g/h.

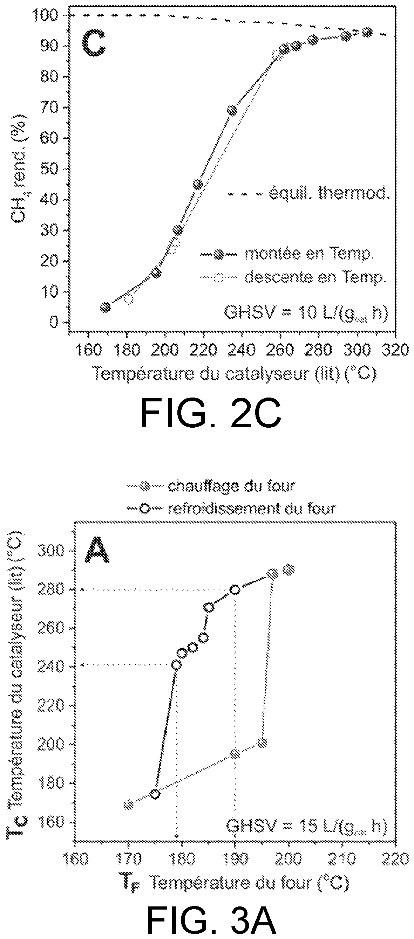

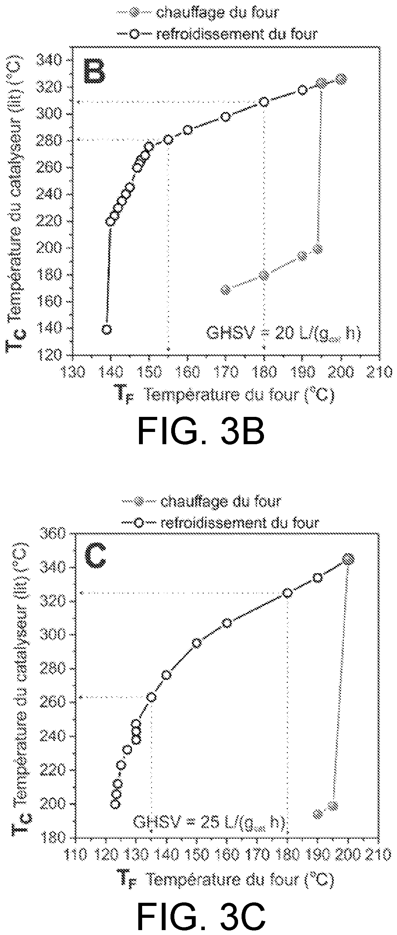

Les figures 3A à 3D représentent la variation de la température du lit catalytique en fonction de la température du four obtenue lors de tests avec un catalyseur Ni10/UO$_{2+x}$ en présence d'un mélange gazeux de rapport molaire $H_2/CO_2$ de 4, pour une vitesse spatiale horaire de gaz de 15, 20, 25 et 30 L/g/h.

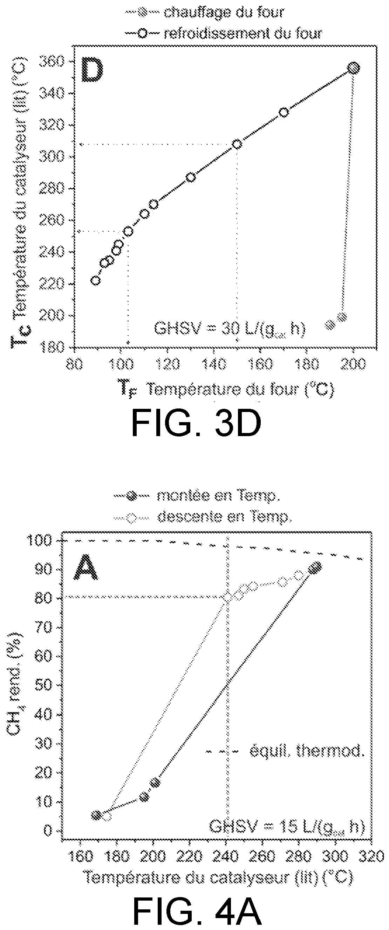

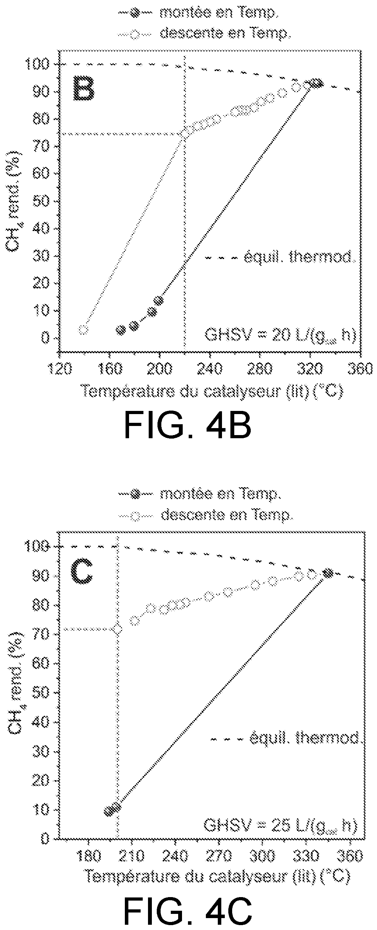

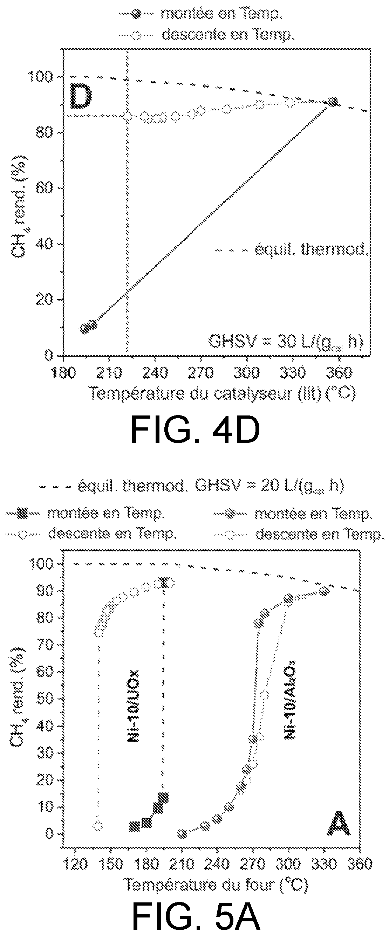

Les figures 4A à 4D représentent le rendement en méthane en fonction de la température du lit catalytique obtenu lors de tests avec un catalyseur Ni10/UO$_{2+x}$ en présence d'un mélange gazeux de rapport molaire $H_2/CO_2$ de 4, pour une vitesse spatiale horaire de gaz de 15, 20, 25 et 30 L/g/h.
Les figures 5A à 5C représentent respectivement le rendement en méthane en fonction de la température du four, la variation de la température du lit catalytique en fonction de la température du four et le rendement en méthane en fonction de la température du lit catalytique obtenus lors de tests avec les catalyseurs Ni10/UO$_{2+x}$ et Ni10/Al$_2$O$_3$ en présence d'un mélange gazeux de rapport molaire $H_2/CO_2$ de 4, avec une vitesse spatiale horaire de gaz de 20 L/g/h.

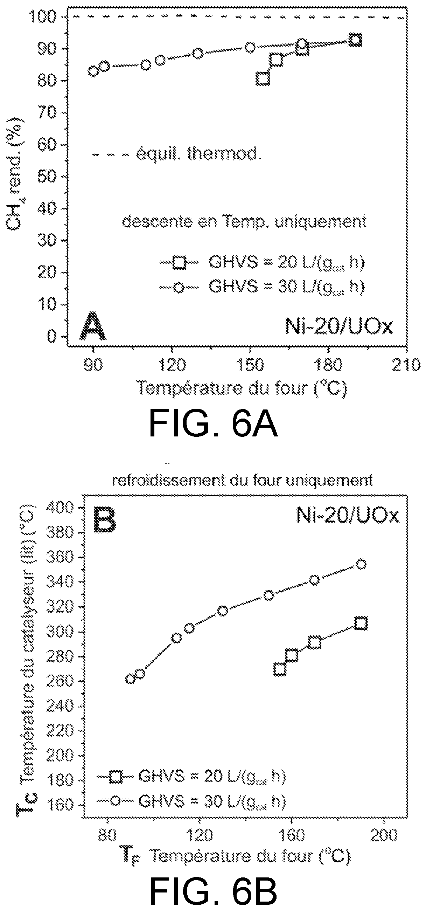

Les figures 6A à 6C présentent le rendement en méthane en fonction de la température du lit catalytique obtenu lors de tests avec un catalyseur Ni20/UO$_{2+x}$ en présence d'un mélange gazeux de rapport molaire $H_2/CO_2$ de 4, avec une vitesse spatiale horaire de gaz de 20 L/g/h.

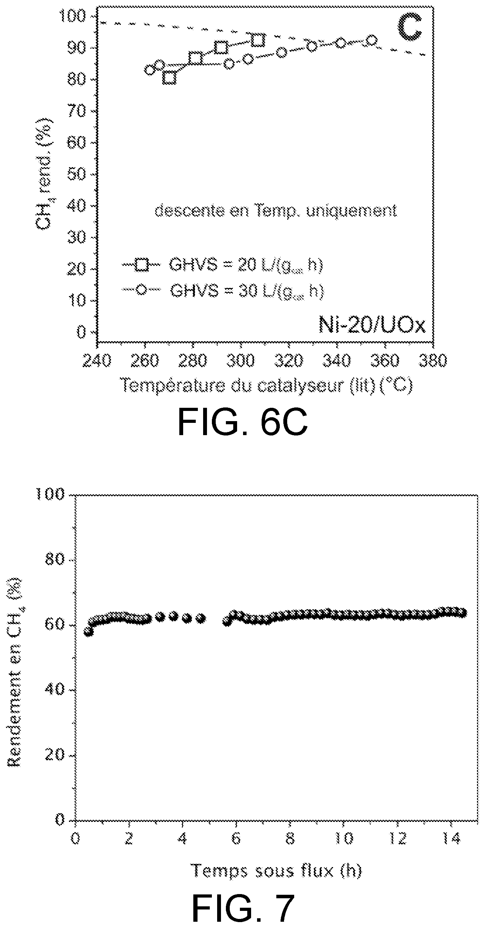

La figure 7 illustre le rendement en méthane en fonction du temps pour un test catalytique réalisé avec un catalyseur Ni20/UO$_{2+x}$, en présence d'un mélange gazeux de rapport molaire $H_2/CO_2$ de 4 et avec une vitesse spatiale horaire de gaz de 20 L/g/h, dans lequel le lit catalytique a été chauffé par induction au moyen d'une bobine.

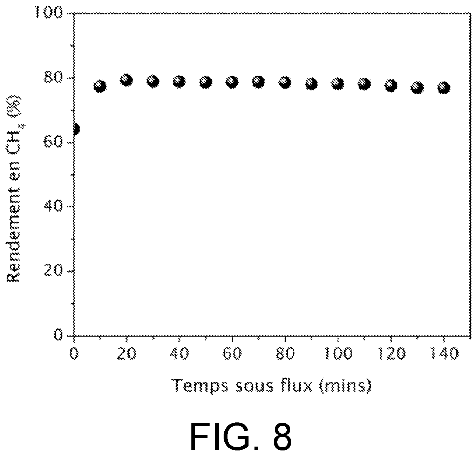

La figure 8 illustre le rendement en fonction lors d'une conversion du $CO_2$ en méthane en mode « auto-méthanation » en présence d'un catalyseur Ni20/UO$_{2+x}$ avec un mélange gazeux de rapport molaire $H_2/CO_2$ de 4, sous une vitesse spatiale horaire de gaz de 20 L/g/h, dans lequel le lit catalytique a été préalablement chauffé par induction au moyen d'une bobine.

La figure 1A est une représentation du dispositif micropilote utilisé pour l'étude de la réaction catalytique de conversion du $CO_2$. Le dispositif 1 comporte une enceinte en verre 2 de diamètre interne DI de 6 mm contenant un lit de catalyseur 3 compris entre une couche supérieure 4 et une couche inférieure 5 de laine de quartz. L'enceinte 2, comprenant une entrée 6 des gaz réactifs et une sortie 7 de l'effluent, est reçue dans un four 8 équipé de structures de chauffe 9 entourant la paroi du four.

Le dispositif est par ailleurs équipé d'un premier thermocouple 10 placé dans la paroi du four et d'un second thermocouple 11 plongeant dans le lit catalytique permettant de suivre respectivement le profil de température du four $T_F$ et celui du lit catalytique $T_C$ pendant les tests.

Le micropilote est alimenté par des bonbonnes de gaz : hydrogène, dioxyde de carbone et argon comme gaz de purge. Les débits de gaz sont mesurés et régulés par des débitmètres massiques couplés avec des électrovannes. La température des gaz entrant est ajustée par passage à travers un préchauffeur, dont la température peut être régulée jusqu'à 400 °C, avant d'alimenter le réacteur catalytique. Le mode de fonctionnement décrit ici est prévu pour une réaction de méthanation sous chauffage indirect par effet Joule.

L'effluent gazeux récupéré en sortie de l'enceinte du réacteur est refroidi *via* un condenseur en verre puis un condenseur à effet Peltier (T ≈ 10 °C). L'eau est donc considérée comme intégralement condensée lorsque l'effluent gazeux de sortie est analysé.

La pression opératoire dans le micropilote est régulée par une vanne de régulation située en aval du condenseur.

L'effluent gazeux sec est ainsi prélevé en aval de la vanne de régulation et analysé au moyen d'un micro-chromatographe en phase gazeuse (R3000, SRA Instrument) qui est équipé de deux colonnes différentes : une colonne de tamis moléculaire (MS5A) permettant la séparation de CO, $CH_4$ et $H_2$ et une colonne d'adsorbant polymère (PPU) permettant entre autres l'analyse du $CO_2$, du méthane, de l'éthane, de l'éthylène et de l'acétylène.

Pour la conduite des essais de conversion catalytique, l'hydrogène est toujours introduit dans le réacteur avant le dioxyde de carbone. Lors du changement de la température de réaction les conditions opératoires sont maintenues jusqu'à la stabilisation de la température au sein du réacteur et de la composition du mélange gazeux en sortie du réacteur. Le temps nécessaire à cette stabilisation est d'environ 40 minutes. À partir de l'état stationnaire des conditions opératoires, la réaction catalytique est maintenue ensuite pendant au moins une heure.

À la fin des tests, la température du réacteur est abaissée jusqu'à la température ambiante sous un flux d'argon avant de décharger le catalyseur. L'analyse de la composition du gaz sec et la mesure de son débit permettent de calculer le taux de conversion du $CO_2$ et la sélectivité en $CH_4$ obtenus.

Le taux de conversion du $CO_2$ est défini comme suit :

$$X_{CO2} = \left( 1 - \frac{D\acute{e}bit_{sortie} * A_{CO2,sortie}}{D\acute{e}bit_{entr\acute{e}e} * A_{CO2,entr\acute{e}e}} \right) * 100$$

avec :

- $A_{CO2, entr\acute{e}e}$ = aire du pic du $CO_2$ du flux gazeux en entrée du réacteur mesurée par le micro-chromatographe ;
- $A_{CO2, sortie}$ = aire du pic du $CO_2$ de l'effluent gazeux en sortie du réacteur mesurée par le micro-chromatographe ;
- Débit sortie = débit de l'effluent gazeux en sortie du réacteur ;
- Débit entrée = débit du flux gazeux ($H_2$+$CO_2$) entrant dans le réacteur.

Lorsque la sélectivité en méthane est égale à 100 %, le taux de conversion du $CO_2$ peut être calculé d'après l'équation suivante :

$$X_{CO2} = X_{CH4} = \frac{x_{CH4,sortie}}{x_{CH4,sortie} + x_{CO2,sortie}} * 100,$$

$X_{CH4,sortie}$ étant la concentration molaire du méthane dans l'effluent.

La relation ci-dessus peut également s'écrire, en considérant que l'eau formée par la réaction est complètement condensée dans le condensateur de sortie et qu'il n'y a pas de formation de CO :

$$X_{CH4} = X_{CO2} = \frac{(1 + R).x_{CH4,sortie}}{(1 + 4).x_{CH4,sortie}} * 100,$$

R étant le rapport molaire $H_2$/$CO_2$ dans le mélange réactionnel.

La dernière équation utilise seulement un paramètre expérimental $x_{CH4,sortie}$ qui est la concentration molaire de méthane formé et dont l'incertitude relative est la plus faible (moins de 5 %).

Dans la mesure où la sélectivité en méthane est de 100 %, le rendement en méthane de la réaction est égal au taux de conversion du $CO_2$ déterminé par les formules ci-dessus.

Les catalyseurs selon l'invention ont été préparés à partir d'une poudre d'$U_3O_8$ (Prolabo) qui est mise en forme par sphéronisation en présence d'eau distillée.

Les billes obtenues sont ensuite imprégnées avec une solution aqueuse de nitrate de nickel par la technique d'imprégnation à sec. Le volume poreux total des billes d'$U_3O_8$ a été déterminé par une imprégnation à sec d'une solution d'eau distillée.

L'incorporation du nickel sur le support par imprégnation à partir d'une solution aqueuse de nitrate de nickel a été réalisée en une seule étape pour fournir des catalyseurs dont la teneur massique est 10 %, 15 % et 20 % de nickel par rapport à la masse totale de catalyseur.

À l'issue de l'étape d'imprégnation ou des étapes d'imprégnation, le solide est séché à l'air pendant 3 heures puis calciné sous air dans un réacteur tubulaire étanche à 350 °C pendant 2 heures.

Les billes calcinées sont tamisées afin de ne récupérer que la fraction ayant une dimension de grain située entre 0,2 mm et 0,8 mm.

Avant d'être utilisées dans la réaction de conversion, les billes calcinées sont activées par réduction sous flux d'hydrogène pur (50 mL/min) à 350 °C pendant deux heures.

L'analyse de diffraction des rayons X des catalyseurs après réduction sous hydrogène indique la présence d'une phase $UO_2$ majoritaire, d'$U_3O_8$ minoritaire et du nickel métal.

**EXEMPLES**

Dans les exemples qui suivent, les vitesses spatiales horaires sont exprimées par rapport à la quantité de catalyseur mise en oeuvre.

**Exemple 1 : Évaluation du catalyseur Ni10/UO$_{2+x}$ à une vitesse spatiale horaire de gaz de 10 L/g/h sous chauffage indirect par effet Joule**

L'activité catalytique du catalyseur Ni (10 % massique) sur un support $UO_{2+x}$ préparé selon la méthode décrite ci-dessus.

400 mg de catalyseur ont été préalablement mélangés avec du carbure de silicium (SiC) comme diluant inerte. Le mélange est introduit dans le réacteur en verre et forme un lit catalytique d'environ 12 mm d'épaisseur.

Les tests ont été réalisés avec un mélange gazeux dont le ratio $H_2/CO_2$ (mol/mol) est égal à 4 pour une vitesse spatiale horaire de gaz de 10 L/g/h, à une pression de 0,1 MPa et en suivant la température du four $T_F$ et celle du lit catalytique $T_C$.

En référence à la figure 2A, on constate que la conversion du $CO_2$ reste faible pour des températures du four $T_F$ inférieures à 197 °C. Au-delà de cette valeur, la réaction catalytique démarre brusquement et la conversion du $CO_2$ déterminée est d'environ 90 % pour une température $T_F$ de 200 °C. Au-delà de 200 °C, le taux de conversion du $CO_2$ atteint une asymptote qui tend vers la valeur de 95 % pour une température supérieure à 200 °C, proche de l'équilibre thermodynamique.

En référence à la figure 2B qui représente la variation de la température dans le lit catalytique $T_C$ en fonction de la température du four $T_F$, on note que lorsque la température du four $T_F$ atteint 197 °C, la température mesurée du lit catalytique $T_C$ est de l'ordre de 270 °C. Cette différence de température $\Delta T$ ($T_C$-$T_F$) d'environ 70 °C correspond à la chaleur dégagée par l'exothermicité de la réaction qui ne peut pas être évacuée du lit catalytique par les échanges avec le flux gazeux. Cet écart de température permet de comprendre le saut de conversion du $CO_2$ observé sur la figure 2A.

Un suivi de la réaction catalytique a été par ailleurs effectué au cours du processus de refroidissement du réacteur en mesurant la conversion du $CO_2$ en fonction de la température dans le lit catalytique $T_C$. Le refroidissement a été opéré en diminuant la température de consigne du four, tout en maintenant l'injection du flux gazeux $H_2$ et $CO_2$. L'objectif est de déterminer si une hystérésis dans le processus catalytique est observable lors de la diminution de la température du four $T_F$, qui se traduirait par un maintien de la conversion du $CO_2$ grâce uniquement à la chaleur dégagée par la réaction.

En référence à la figure 2C qui donne le rendement en $CH_4$ en fonction de la température mesurée dans le lit catalytique $T_C$ lors de la montée et la descente en température dans le lit catalytique, on constate que la réaction exothermique dégage une forte chaleur dans le lit catalytique. Néanmoins, dans ces conditions réactionnelles, la chaleur dégagée au sein du lit catalytique est insuffisante pour permettre d'atteindre un état d'équilibre entre la chaleur produite par la réaction et celle qui est évacuée par l'effluent gazeux en sortie de réacteur. En effet, lors de la descente lente de la température de consigne du four $T_F$ la conversion de $CO_2$ chute avec la température Tc. On n'observe pas d'hystérésis catalytique dans les conditions opératoires de l'exemple 1.

**Exemple 2 : Évaluation du catalyseur Ni10/UO$_{2+x}$ à des vitesses spatiales horaires de gaz de 15, 25 et 30 L/g/h sous chauffage indirect par effet Joule**

Les tests de l'exemple 2 ont été réalisés avec la même configuration de réacteur que celle de l'exemple 1 afin d'étudier le comportement catalytique du catalyseur contenant 10 % en masse de Ni par rapport à la masse totale de catalyseur, à des vitesses spatiales horaires de gaz plus élevées au cours des phases de montée et de descente en température du four. Ces essais permettent notamment de déterminer vérifier si une hystérésis (écart de conversion lors de la montée et de la descente de température) peut être observée.

Les figures 3A à 3D représentent l'évolution de la température du lit catalytique $T_C$ en fonction de la température du four $T_F$ au cours de la phase de montée et de descente en température du four pour les différentes valeurs de vitesse spatiale horaire de gaz égales à 15, 20, 25 et 30 L/g/h, en présence d'un flux gazeux contenant un ratio molaire $H_2/CO_2$ égal à 4.

Quant aux figures 4A à 4D, elles indiquent le rendement en méthane, représentative de l'activité en méthanation du catalyseur, en fonction de la température du lit catalytique et aux différentes vitesses spatiales horaires de gaz lors des phases de montée et de descente en température du four.

Avec le catalyseur Ni10/UO$_{2+x}$ et pour une vitesse spatiale horaire de gaz supérieure ou égale à 15 L/g/h, une hystérésis apparaît lors de la diminution de la température du four. Ce phénomène est d'autant plus notable que les vitesses spatiales horaires de gaz sont élevées (figures 3A à 3D). En effet, l'activité en méthanation diminue d'une manière progressive mais cependant demeure encore élevée pour des températures mesurées dans le lit catalytique supérieures à 200 °C.

On observe en outre que la température maximum mesurée dans le lit catalytique $T_C$ augmente avec la vitesse spatiale horaire et que la chute de la température dans le lit catalytique pendant la descente en température du four est d'autant moins rapide que la vitesse spatiale horaire de gaz est grande. Ceci traduit donc que la chaleur générée par la conversion élevée du $CO_2$ permet de maintenir en température le lit catalytique (figures 4A à 4D).

Dans le cas du test réalisé à une vitesse spatiale horaire de gaz de 30 L/g/h (figure 3D), il a été remarqué que, même lorsque le four a été éteint, la température affichée par le thermocouple du four est restée au-dessus de 90 °C, indiquant qu'une partie de la chaleur générée dans le lit catalytique (dont la température est maintenue à environ 220 °C grâce à l'exothermicité de la réaction) contribue à maintenir le four en température grâce aux échanges de chaleur.

Le système catalytique selon l'invention est capable de fonctionner en mode « auto-méthanation » lorsque la vitesse spatiale horaire de gaz est d'au moins 25 L/g/h (soit 25 m$^3$/kg/h) et tant que la température du lit catalytique reste à une valeur supérieure ou égale à 200 °C, grâce à l'établissement d'un équilibre entre la chaleur dégagée par la réaction et la chaleur échangée avec le flux de réactifs.

**Exemple 3 (comparatif)** : **Évaluation du catalyseur Ni10/gamma-Alumine par rapport au catalyseur Ni10/UO$_{2+x}$ à 20 L/g/h sous chauffage indirect par effet Joule**

Des tests comparatifs ont été effectués dans les mêmes conditions opératoires que celles de l'exemple 1, c'est-à-dire avec un mélange gazeux dont le ratio molaire $H_2/CO_2$ est de 4 mais en présence d'un catalyseur comprenant une teneur massique en nickel sur un support de type alumine ($Al_2O_3$).

Le catalyseur comparatif a été préparé de la manière suivante : le support à base de gamma-$Al_2O_3$, noté par la suite alumine, est sous forme d'extrudés (1 mm de diamètre et 3 mm de longueur, Ketjen 300B fournie par Akzo Nobel) est imprégné à sec avec une solution aqueuse de nitrate de nickel à température ambiante. Le matériau est laissé maturer à température ambiante pendant 3 heures puis séché à 110 °C sous air pendant 3 heures afin d'éliminer le solvant. Le matériau sec est calciné sous air à 350 °C pendant 2 heures (avec une pente de montée en température de 3 °C/min). Le précurseur de catalyseur ainsi obtenu est réduit directement dans le réacteur catalytique sous un flux d'hydrogène pur (20 mL/min) à 350 °C pendant 2 heures.

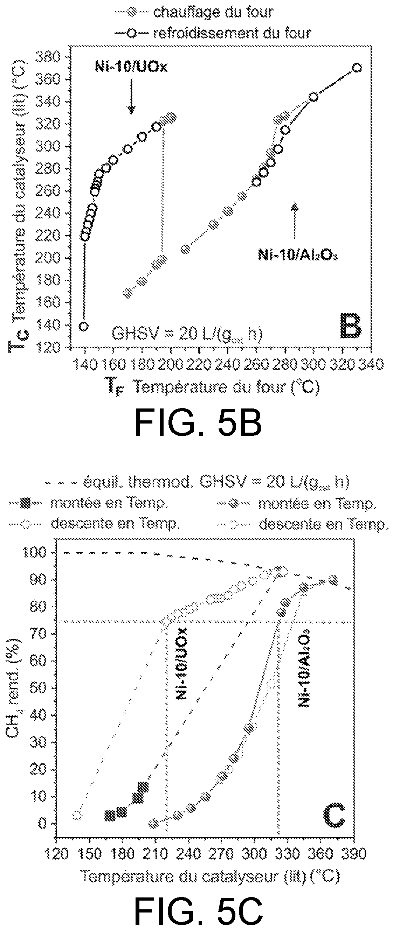

Les résultats expérimentaux sont reportés sur les figures 5A et 5C qui donnent le rendement en méthane en fonction de la température du four $T_F$ et du lit catalytique Tc lors des phases de montée et de descente en température du four. La figure 5A confirme l'excellence activité en méthanation du catalyseur Ni10/UO$_{2+x}$ par rapport à son homologue Ni10/Al$_2O_3$.

La figure 5B présente l'évolution de la température dans le lit catalytique $T_C$ en fonction de la température du four $T_F$ au cours des phases de montée et de descente en température du four. Pour le catalyseur Ni10/UO$_{2+x}$, lorsque la température du four dépasse 190 °C, un saut important de la température dans le lit catalytique est mesuré (320 °C). Ce saut est expliqué par une conversion plus élevée du $CO_2$ en $CH_4$. Dans le cas du catalyseur Ni10/Al$_2O_3$, on observe un très faible écart entre les deux températures, ce qui traduit une plus faible conversion.

En référence à la figure 5C, on constate qu'à iso-rendement en $CH_4$, la température dans le lit catalytique Ni10/Al$_2O_3$ est toujours supérieure à celle mesurée dans le lit catalytique Ni10/UO$_{2+x}$. Par exemple, comme indiqué sur la figure 5C, pour un rendement en $CH_4$ de 75 %, la température mesurée dans le lit catalytique contenant du catalyseur Ni10/Al$_2O_3$ est de 320 °C alors que celle mesurée dans le lit catalytique contenant le catalyseur Ni10/UO$_2$ est de 220 °C. Ces résultats confirment donc la forte réactivité du catalyseur Ni10/UO$_{2+x}$.

La moindre activité catalytique du catalyseur Ni10/Al$_2O_3$ se manifeste également par le fait que l'activité catalytique n'est pas maintenue lors de la descente en température du four et donc par l'absence d'hystérésis dans le profil de température.

**Exemple 4 : Évaluation du catalyseur Ni20/UO$_{2+x}$ à des vitesses spatiales horaires de gaz de 20 L/g/h et 30 L/g/h sous chauffage indirect par effet Joule**

Des tests ont été réalisés avec un catalyseur comprenant 20 % en masse de nickel par rapport à la masse totale de catalyseur sur un support UO$_{2+x}$. Ce catalyseur a été préparé selon le même protocole que celui de l'exemple 1 avec une double imprégnation à sec d'une solution aqueuse de nitrate de nickel.

Le catalyseur après réduction sous hydrogène a été testé sous deux vitesses spatiales horaires de gaz de 20 L/g/h et 30 L/g/h et avec un mélange gazeux de ratio molaire H$_2$/CO$_2$ de 4.

Les figures 6A à 6C montrent respectivement, pour la phase de descente en température du four, le rendement en CH$_4$ en fonction de la température du four T$_F$, la variation de la température du lit catalytique en fonction de la température du four et enfin le rendement en CH$_4$ en fonction de la température du lit catalytique T$_C$.

On peut observer que le catalyseur selon l'invention contenant 20 % en masse de Ni présente une excellente activité catalytique. Le rendement en CH$_4$ reste supérieur à 80 % pour une gamme de température dans le lit catalytique comprise entre 260 °C et 360 °C. En référence aux figures 6A, 6B et 6C, on note également que le catalyseur est capable de fonctionner selon le mode d'auto-méthanation pendant la descente en température du four. Par exemple, pour une vitesse spatiale horaire de gaz de 30 L/g/h et une température de 90 °C mesurée dans le four, le rendement en CH$_4$ reste supérieur à 80 % grâce au maintien en température du lit catalytique à une valeur d'environ 260 °C.

**Exemple 5 : Évaluation du catalyseur Ni20/UO$_{2+x}$ à basse température et vitesses spatiales horaires de gaz de 20 L/g/h sous chauffage inductif**

Des tests ont été réalisés avec un catalyseur comprenant 20 % en masse de nickel par rapport à la masse totale du catalyseur sur un support UO$_{2+x}$.

Le dispositif 12 mis en oeuvre pour réaliser le chauffage est représenté à la figure 1B.

Le lit catalytique 13, qui est contenu dans un réacteur 14 en verre de 8 mm de diamètre interne, comporte une couche 15 constituée de 800 mg de catalyseur mélangés avec 200 mg de carbure de silicium en tant que diluant thermique (SiC). Le mélange catalyseur et SiC est déposé au-dessus d'un feutre de graphite 16 jouant le rôle de suscepteur. L'épaisseur du lit catalytique 13, qui est compris entre deux couches de laine de quartz 17, 18, mesure approximativement 17 mm.

Le réacteur 14 est chauffé au moyen d'un système de chauffage par induction EasyHeat 8310, 10 kW commercialisé par la société Ambrell Ltd. Ce système est équipé d'une bobine d'induction en cuivre 19 à six spires qui est refroidie par de l'eau circulant à l'intérieur de la bobine. Le réacteur est disposé à l'intérieur de la bobine de sorte que le lit catalytique soit encerclé par la bobine d'induction. Le contrôle de la température en temps réel est assuré par un régulateur Eurotherm 3504 connecté à un pyromètre laser Optris™ pointant sur le lit catalytique.

Après une étape de réduction *in situ* du catalyseur à 300 °C pendant 30 min sous H$_2$, ce dernier est laissé refroidir à 160 °C sous Ar. Le mélange réactionnel (1 mol de CO$_2$ pour 4 mol de H$_2$) est introduit dans le réacteur avec une vitesse spatiale horaire de gaz de 20 L/g/h.

Le lit catalytique est chauffé progressivement par induction jusqu'à une température de 190 °C mesurée par le pyromètre laser. Au cours du test, la température dans le lit catalytique est fixée à la température de consigne de 190 °C.

Les résultats sont présentés sur la figure 7. On constate que le catalyseur Ni20/UO$_{2+x}$ présente un rendement en CH$_4$ stable à environ 62 %, pour une température dans le lit catalytique de 190 °C. Ces résultats indiquent que le catalyseur Ni20/UO$_2$ permet de réaliser la réaction de méthanation à une température relativement basse par rapport aux résultats reportés dans la littérature. De plus, l'induction ne chauffe spécifiquement que le catalyseur solide et le suscepteur et non pas les réactifs gazeux passant à travers le lit catalytique d'où un gain non négligeable en termes d'apport énergétique au procédé. Il est à noter que le catalyseur ne présente pas de désactivation pendant la durée du test confirmant ainsi l'excellente stabilité de ce dernier.

**Exemple 6 : Évaluation du catalyseur Ni20/UO$_{2+x}$ à des vitesses spatiales horaires de gaz de 20 L/g/h sous chauffage inductif**

Des tests ont été menés avec le même catalyseur et la même configuration de réacteur que ceux de l'exemple 5.

L'exemple 6 se différencie de l'exemple 5 par les conditions de mise en température du lit catalytique. Le lit catalytique est chauffé par induction progressivement jusqu'à la température de consigne de 170 °C avec un courant appliqué à l'inducteur de 120 ampères. Puis on augmente brusquement la température de consigne à 190 °C qui s'accompagne d'un saut de température au sein du lit qui atteint 230 °C. Cette exothermicité dans le lit catalytique traduit un emballement de la réaction catalytique de conversion du CO$_2$. Le courant appliqué à l'inducteur est ensuite diminué jusqu'à 23 ampères puis totalement coupé et enfin la bobine d'induction est déplacée de telle sorte que le lit catalytique se trouve hors de son environnement.

Les résultats de la conversion du $CO_2$ en méthane après arrêt de l'inducteur sont présentés sur la figure 8. Le catalyseur selon l'invention réalise une conversion d'environ 80 % du $CO_2$ en $CH_4$ en mode auto-méthanation (sans apport externe de chaleur) pendant 140 min. Lors de cet essai, il a été également noté que la température mesurée dans le lit catalytique oscille entre 220 °C et 230 °C grâce à la chaleur dégagée par la réaction catalytique.

**Revendications**

1. Procédé de conversion du $CO_2$ en méthane, dans lequel :

   - on met en contact de l'hydrogène et une charge gazeuse comprenant du $CO_2$ dans au moins un réacteur de méthanation comprenant un lit de catalyseur à une température dans le lit catalytique comprise entre 160 °C et 550 °C, à une pression comprise entre 0,1 MPa et 1 MPa, avec une vitesse spatiale horaire de gaz comprise entre 10 m$^3$/kg/h et 50 m$^3$/kg/h et avec un rapport molaire $H_2/CO_2$ compris entre 1 et 8 ;
   - le catalyseur contient du Ni métal déposé sur un support en oxyde d'uranium de formule $UO_{2+x}$, avec x étant compris entre 0,01 et 0,6, et dont la teneur massique en nickel est comprise entre 5 % et 40 % de nickel métal par rapport à la masse totale du catalyseur ; et dans lequel :

      - le catalyseur est préparé par un procédé comprenant les étapes a) à c) suivantes :

         a) on imprègne un précurseur de support constitué essentiellement d'un oxyde d'uranium (IV) et/ou d'uranium (VI) avec une solution contenant un précurseur de nickel et un solvant polaire ;
         b) on calcine sous air et à une température d'au moins 250 °C le précurseur de support imprégné ; et
         c) on réduit le précurseur de support imprégné et calciné sous hydrogène à une température d'au moins 300 °C.

2. Procédé selon la revendication 1, dans lequel, avant l'étape de calcination, on sèche le précurseur de support imprégné à une température inférieure à 200 °C.

3. Procédé selon la revendication 1 ou la revendication 2, dans lequel le précurseur de support est choisi parmi l'$U_3O_8$, l'$UO_2$, l'$UO_4$ et l'$UO_3$.

4. Procédé selon l'une des revendications 1 à 3, dans lequel le précurseur de support est sous forme d'extrudés cylindriques ou multilobés, de sphères, d'anneaux, de monolithes en structure de nid d'abeille ou d'une poudre.

5. Procédé selon l'une des revendications 1 à 4, dans lequel le précurseur de nickel est choisi parmi l'hydroxyde, l'hydroxycarbonate, le carbonate et le nitrate de nickel.

6. Procédé selon l'une des revendications 1 à 5, dans lequel l'étape a) d'imprégnation met en oeuvre une imprégnation à sec ou en excès.

7. Procédé selon l'une des revendications 1 à 6, dans lequel l'étape c) de réduction est réalisée de manière *in situ* dans le réacteur de méthanation.

8. Procédé selon l'une des revendications 1 à 7, dans lequel la charge gazeuse comprenant du $CO_2$ et l'hydrogène sont envoyés séparément ou en mélange dans le réacteur de méthanation selon une direction descendante.

9. Procédé selon l'une des revendications 1 à 8, dans lequel le réacteur de méthanation est un réacteur adiabatique.

10. Procédé selon l'une des revendications 1 à 9, dans lequel le réacteur de méthanation est un réacteur à lit fixe ou fluidisé de catalyseur.

11. Procédé selon la revendication 10, dans lequel le lit de catalyseur est un lit fixe et on soumet le lit de catalyseur à un champ électromagnétique alternatif de manière à chauffer le lit de catalyseur par induction.

12. Procédé selon la revendication 11, dans lequel le lit fixe de catalyseur comprend en outre des particules suscepteurs.

13. Procédé selon l'une des revendications 1 à 12, dans lequel on opère le procédé avec une source d'énergie renouvelable.

14. Procédé selon l'une des revendications 1 à 13, dans lequel le réacteur est un réacteur à lit fixe de catalyseur et dans lequel on fixe la vitesse spatiale horaire de gaz à au moins 15 m$^3$/kg/h de manière à maintenir une température du lit de catalyseur $T_C$ à au moins 200 °C, moyennant quoi on opère la réaction de conversion sans apport de chaleur externe.

15. Procédé de préparation d'un catalyseur de méthanation du $CO_2$ comprenant du nickel métal déposé sur un support en oxyde d'uranium de formule $UO_{2+x}$ avec x étant compris entre 0,01 et 0,6, et dans lequel la teneur massique de nickel est comprise entre 5 % et 40 % de nickel métal par rapport à la masse totale du catalyseur, lequel procédé comprend les étapes a) à c) suivantes :

a) on imprègne un précurseur de support constitué essentiellement d'un oxyde d'uranium (IV) et/ou d'uranium (VI) avec une solution contenant un précurseur de nickel et un solvant polaire ;
b) on calcine sous air et à une température d'au moins 250 °C le précurseur de support imprégné ; et
c) on réduit le précurseur de support imprégné et calciné sous hydrogène à une température d'au moins 300 °C.


**Patentansprüche**

1. Verfahren zur Umsetzung von $CO_2$ in Methan, wobei:

- Wasserstoff und eine $CO_2$ umfassende Gasladung in Kontakt gebracht werden in mindestens einem Methanisierungsreaktor, der ein Katalysatorbett umfasst, bei einer Temperatur im katalytischen Bett zwischen 160 °C und 550 °C, bei einem Druck zwischen 0,1 MPa und 1 MPa, mit einer stundenbezogenen Gasraumgeschwindigkeit zwischen 10 m$^3$/kg/h und 50 m$^3$/kg/h und mit einem Molverhältnis $H_2$/$CO_2$ zwischen 1 und 8;
- der Katalysator Metall Ni enthält, das auf einem Träger aus Uranoxid der Formel $UO_{2+x}$ abgeschieden ist, wobei x zwischen 0,01 und 0,6 liegt, und dessen Massengehalt an Nickel zwischen 5 % und 40 % Nickelmetall im Verhältnis zur Gesamtmasse des Katalysators liegt; und wobei:

- der Katalysator durch ein Verfahren zubereitet wird, das die folgenden Schritte a) bis c) umfasst:

a) Imprägnieren einer Vorstufe des Trägers, die im Wesentlichen aus einem Uranoxid (IV) und/oder aus Uran (VI) besteht, mit einer Lösung, die eine Vorstufe von Nickel und ein polares Lösungsmittel enthält;
b) Kalzinieren mittels Luft und bei einer Temperatur von mindestens 250 °C der imprägnierten Vorstufe des Trägers; und
c) Reduzieren der imprägnierten und kalzinierten Vorstufe des Trägers mittels Wasserstoff, bei einer Temperatur von mindestens 300 °C.

2. Verfahren nach Anspruch 1, wobei vor dem Kalzinierungsschritt die imprägnierte Vorstufe des Trägers bei einer Temperatur unter 200 °C getrocknet wird.

3. Verfahren nach Anspruch 1 oder Anspruch 2, wobei die Vorstufe des Trägers aus $U_3O_8$, $UO_2$, $UO_4$ und $UO_3$ ausgewählt ist.

4. Verfahren nach einem der Ansprüche 1 bis 3, wobei die Vorstufe des Trägers in Form von zylindrischen oder multilobären Extrudierungen, Kugeln, Ringen, Monolithen in Wabenstruktur oder Pulver vorliegt.

5. Verfahren nach einem der Ansprüche 1 bis 4, wobei die Vorstufe von Nickel aus Hydroxid, Hydroxycarbonat, Carbonat und Nickelnitrat ausgewählt ist.

6. Verfahren nach einem der Ansprüche 1 bis 5, wobei der Imprägnierungsschritt a) eine Trocken- oder Überschussimprägnierung implementiert.

7. Verfahren nach einem der Ansprüche 1 bis 6, wobei der Reduzierungsschritt c) *in situ* im Methanisierungsreaktor durchgeführt wird.

8. Verfahren nach einem der Ansprüche 1 bis 7, wobei die $CO_2$ umfassende Gasladung und der Wasserstoff getrennt oder vermischt in einer Abwärtsrichtung in den Methanisierungsreaktor geleitet werden.

9.  Verfahren nach einem der Ansprüche 1 bis 8, wobei der Methanisierungsreaktor ein adiabatischer Reaktor ist.

10. Verfahren nach einem der Ansprüche 1 bis 9, wobei der Methanisierungsreaktor ein Reaktor mit festem oder verwirbeltem Katalysatorbett ist.

11. Verfahren nach Anspruch 10, wobei das Katalysatorbett ein festes Bett ist und das Katalysatorbett einem elektromagnetischen Wechselfeld ausgesetzt wird, um das Katalysatorbett durch Induktion zu erwärmen.

12. Verfahren nach Anspruch 11, wobei das feste Bett des Katalysators ferner Suszeptorpartikel umfasst.

13. Verfahren nach einem der Ansprüche 1 bis 12, wobei das Verfahren mit einer erneuerbaren Energiequelle durchgeführt wird.

14. Verfahren nach einem der Ansprüche 1 bis 13, wobei der Reaktor ein Reaktor mit festem Katalysatorbett ist und wobei die stundenbezogene Gasraumgeschwindigkeit auf mindestens 15 m$^3$/kg/h festgelegt wird, um eine Temperatur des Katalysatorbetts $T_C$ bei mindestens 200 °C zu halten, wodurch die Umsetzungsreaktion ohne externe Wärmezufuhr durchgeführt wird.

15. Verfahren zur Zubereitung eines Katalysators der Methanisierung von $CO_2$, der Nickelmetall umfasst, das auf einem Träger aus Uranoxid der Formel $UO_{2+x}$ abgeschieden ist, wobei x zwischen 0,01 und 0,6 liegt und wobei der Massengehalt an Nickel zwischen 5 % und 40 % Nickelmetall im Verhältnis zur Gesamtmasse des Katalysators liegt, wobei das Verfahren die folgenden Schritte a) bis c) umfasst:

    a) Imprägnieren einer Vorstufe des Trägers, die im Wesentlichen aus einem Uranoxid (IV) und/oder aus Uran (VI) besteht, mit einer Lösung, die eine Vorstufe von Nickel und ein polares Lösungsmittel enthält;
    b) Kalzinieren mittels Luft und bei einer Temperatur von mindestens 250 °C der imprägnierten Vorstufe des Trägers; und
    c) Reduzieren der imprägnierten und kalzinierten Vorstufe des Trägers mittels Wasserstoff, bei einer Temperatur von mindestens 300 °C.

**Claims**

1.  A method for converting $CO_2$ into methane, wherein:

    - hydrogen is contacted with a gas feed comprising $CO_2$ in at least one methanation reactor comprising a catalyst bed at a temperature in the catalytic bed comprised between 160°C and 550°C, at a pressure comprised between 0.1 MPa and 1 MPa, with a gas hourly space velocity comprised between 10 m$^3$/kg/h and 50 m$^3$/kg/h and with an $H_2/CO_2$ molar ratio comprised between 1 and 8;
    - the catalyst comprises Ni metal deposited on a uranium oxide support of formula $UO_{2+x}$, with x being comprised between 0.01 and 0.6, and the nickel mass content of which is comprised between 5% and 40% of nickel metal relative to the total mass of the catalyst; and wherein:

      - the catalyst is prepared by a method comprising the following steps a) to c):

        a) a support precursor consisting essentially of a uranium (IV) and/or uranium (VI) oxide is impregnated with a solution comprising a nickel precursor and a polar solvent;
        b) the impregnated support precursor is calcined in air and at a temperature of at least 250°C; and
        a) the impregnated and calcined support precursor is reduced under hydrogen at a temperature of at least 300°C.

2.  The method according to claim 1, wherein, before the calcination step, the impregnated support precursor is dried at a temperature below 200°C.

3.  The method according to claim 1 or claim 2, wherein the support precursor is selected from $U_3O_8$, $UO_2$, $UO_4$ and $UO_3$.

4.  The method according to one of claims 1 to 3, wherein the support precursor is in the form of cylindrical or multilobed extrudates, spheres, rings, monoliths in a honeycomb structure or a powder.

5. The method according to one of claims 1 to 4, wherein the nickel precursor is selected from nickel hydroxide, hydroxycarbonate, carbonate and nitrate.

6. The method according to one of claims 1 to 5, wherein the impregnation step a) implements a dry or excess impregnation.

7. The method according to one of claims 1 to 6, wherein step c) of reduction is carried out *in situ* in the methanation reactor.

8. The method according to one of claims 1 to 7, wherein the gas feed comprising $CO_2$ and hydrogen are sent separately or mixed into the methanation reactor in a downward direction.

9. The method according to one of claims 1 to 8, wherein the methanation reactor is an adiabatic reactor.

10. The method according to one of claims 1 to 9, wherein the methanation reactor is a reactor with a fixed or fluidised catalyst bed.

11. The method according to claim 10, wherein the catalyst bed is a fixed bed and the catalyst bed is subjected to an alternating electromagnetic field so as to heat the catalyst bed by induction.

12. The method according to claim 11, wherein the fixed catalyst bed further comprises susceptor particles.

13. The method according to one of claims 1 to 12, wherein the method is carried out with a renewable energy source.

14. The method according to one of claims 1 to 13, wherein the reactor is a reactor with a fixed catalyst bed and wherein the gas hourly space velocity is fixed at least at 15 m³/kg/h so as to maintain a temperature of the catalyst bed $T_C$ at least at 200°C, whereby the conversion reaction is carried out without the addition of external heat.

15. A method for preparing a $CO_2$ methanation catalyst comprising nickel metal deposited on a uranium oxide support of formula $UO_{2+x}$ with x being comprised between 0.01 and 0.6, and wherein the mass content of nickel is comprised between 5% and 40% of nickel metal relative to the total mass of the catalyst, which method comprises the following steps a) to c):

a) a support precursor consisting essentially of a uranium (IV) and/or uranium (VI) oxide is impregnated with a solution comprising a nickel precursor and a polar solvent;
b) the impregnated support precursor is calcined in air and at a temperature of at least 250°C; and
c) the impregnated and calcined support precursor is reduced under hydrogen at a temperature of at least 300°C.

FIG. 1A

FIG. 1B

FIG. 2A

FIG. 2B

FIG. 2C

FIG. 3A

FIG. 3B

FIG. 3C

FIG. 3D

FIG. 4A

FIG. 4B

FIG. 4C

FIG. 4D

FIG. 5A

FIG. 5B

FIG. 5C

EP 4 313 403 B1

FIG. 6A

FIG. 6B

FIG. 6C

FIG. 7

FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BERRY et al.** *Applied Catalysis A: General*, 1993, vol. 100, 131-143

- *Applied Catalysis A: General*, 1993, vol. 100, 131-143